## Europäisches Patentamt

## European Patent Office

## Office européen des brevets

(19)

(11) Numéro de publication: **0 160 780**
**B1**

# FASCICULE DE BREVET EUROPÉEN

(12)

(45) Date de publication du fascicule du brevet:
24.02.88

(21) Numéro de dépôt: **85101032.2**

(22) Date de dépôt: **03.05.83**

(51) Int. Cl.⁴: **A 61 F 5/01**

(54) **Chaussure orthopédique pour handicapés moteurs des membres inférieurs.**

(30) Priorité: **21.05.82 FR 8208892**

(43) Date de publication de la demande:
**13.11.85 Bulletin 85/46**

(45) Mention de la délivrance du brevet:
**24.02.88 Bulletin 88/8**

(84) Etats contractants désignés:
**AT BE CH DE GB IT LI LU NL SE**

(56) Documents cité:
**GB-A-1 298 930**
**US-A-1 336 695**
**US-A-1 660 721**
**US-A-2 654 365**
**US-A-2 772 674**
**US-A-3 805 773**
**US-A-4 102 337**

**MEDIZINISCH-ORTHOPAEDISCHE TECHNIK, vol. 101, no. 2, mars-avril 1981, pages 39-41, Stuttgart, DE; R.O. NITSCHKE: "Laterale Hüft-Knie-Fuss-Orthesen"**

(73) Titulaire: **Salort, Guy, 219, rue Raymond Losserand, F-75014 Paris (FR)**

(72) Inventeur: **Salort, Guy, 219, rue Raymond Losserand, F-75014 Paris (FR)**

(74) Mandataire: **Peuscet, Jacques, Cabinet Peuscet 68, rue d'Hauteville, F-75010 Paris (FR)**

## Description

La présente demande décrit une chaussure orthopédique qui permet le fonctionnement physiologique de l'appui podal en confortant et en regulant un mouvement de vis interne. Cette chaussure peut notamment être utilisée en combinaison avec l'appareillage externe de station verticale et de marche décrit dans le brevet européen n° 0 066 028.

L'utilisation de cette chaussure en combinaison avec l'appareillage susmentionné est décrite dans la demande de brevet européen 0 095 396 dont la présente constitue une demande divisionnaire.

La présente invention a donc pour objet le produit industriel nouveau que constitue une chaussure orthopédique pour handicapes moteurs d'un (ou des deux) membre(s) inférieur(s), comprenant

- une coque semi-rigide entourant le pied et le cône inférieur jambier, cette coque comportant une ouverture qui s'étend en regard de la loge antéro-externe du cône jambier et en regard du bord externe du pied, au-dessus du cuboïde et d'au moins un rayon métatarsien;

- une armature élastique comportant, d'une part, un levier-ressort lié à la coque et disposé verticalement en regard du péroné, depuis la face externe du calcanéum jusqu'à la zone de bordure supérieure de la coque, et, d'autre part, des moyens élastiques régulateurs implantés à l'extérieur de la coque, en premier lieu à partir du bord interne de la semelle de la coque vers la partie supérieure de l'empeigne et vers la bordure supérieure de la tige de la coque, et, en second lieu, à partir de la zone de la malléole interne jusqu'à la zone postéro-externe du talon de la semelle;

- et des moyens de fermeture de la coque de la chaussure.

Dans un mode préfére de réalisation de la chaussure selon l'invention, les moyens de fermeture de la coque comprennent un moyen élastique tendu entre le bord interne de la semelle de la coque au niveau du premier métatarsien et le bord externe de ladite semelle au niveau du cuboïde; les moyens de fermeture de la coque comprennent un moyen élastique tendu entre le bord interne de la semelle de la coque, au niveau du premier métatarsien, et le bord externe de ladite semelle, au niveau du cinquième metatarsien; en bout du pied, l'ouverture de la coque s'étend transversalement au-dessus des deux derniers métatarsiens; la coque entoure le cône inférieur jambier jusqu'au-dessus du tiers inférieur de la jambe et les moyens de fermeture comprennent une bande de serrage s'étendant tout le long de la bordure supérieure de la tige de la coque; la coque de la chaussure est réalisée d'une seule pièce en matière plastique moulée; la chaussure comprend intérieurement une semelle de soutien du pied, moulée aux reliefs de la voûte plantaire de l'utilisateur mis en position d'équilibre vertical.

Les moyens élastiques régulateurs, qui sont associés au levier-ressort de la coque, régulent les actions dudit levier-ressort. On a constaté que cette architecture particulière de la chaussure permet d'encaisser de légères déformations en mouvements de vis interne et de restituer élastiquement les efforts. Il en résulte une légère flexion physiologique de la cheville ainsi qu'un deroulement du pas podal (talon-bord externe-bord interne).

Lorsque la chaussure orthopédique selon l'invention est utilisée en combinaison avec un appareillage externe selon le brevet européen 0 066 028, la coque de la chaussure est reliée à l'extrémité inférieure du levier-ressort tibial de l'appareillage par un point d'attache situé dans la zone de la malléole interne; ce levier-ressort tibial s'étend entre une extrémité supérieure sensiblement sous-articulaire et une extrémité inferieure sensiblement sus-malléolaire ; ce levier-ressort, lié à la coque de la chaussure et disposé verticalement en regard du péroné, peut être une lame d'acier trempé de 40 mm de large et de 1 mm d'épaisseur environ: il régule les mouvements d'inclinaison interne (roulement et abaissement du calcanéum) et les mouvements de vis interne du segment jambier sur les articulations tibio-tarsienne et sous-astragalienne.

On va décrire maintenant, à titre d'exemple purement illustratif et non limitatif, un mode de realisation de la chaussure orthopédique selon l'invention, cette chaussure etant utilisée en combinaison avec un appareillage externe du type de celui décrit dans le brevet européen 0 066 028.

Sur ce dessin:
- la figure 1 représente, en perspective, le détail d'une chaussure selon l'invention, vue du côté interne;
- la figure 2 représente, en perspective, la chaussure de la figure 1 vue du côté externe.

Sur le dessin, on a représenté le levier-ressort tibial 6 d'un appareillage selon le brevet européen 0 066 028. Ce levier-ressort 6, qui est inséré sur la genouillère en position antéro-interne, s'étend le long de la diaphyse tibiale jusqu'en son extrémité inférieure 6b, au niveau de la malléole interne, où il est fixé sur la tige montante de la chaussure orthopédique 7 selon l'invention.

La chaussure 7 est constituée d'une coque 70 en matière plastique moulée d'une épaisseur de 3 mm, par exemple en polyéthylène basse densité (d = 0,95). Cette coque, réalisée d'une seule pièce, est constituée d'une semelle 701 surmontée par une empeigne, dont la partie supérieure a été designée par 703 et par une tige, dont la bordure supérieure 702 est destinée à se trouver légèrement au-dessus du tiers inférieur de la jambe. La tige et l'empeigne de la coque semi-rigide 70 comportent une ouverture 71, qui s'étend verticalement sur la tige en regard de la loge antéro-externe du cône jambier destiné à y être placé et qui s'étend sur l'empeigne en regard du bord externe du pied au-dessus du cuboïde; l'extrémité avant de cette ouverture 71 est disposée transversalement au-dessus des deux derniers rayons métatarsiens. La coque 70 porte dans la zone de la malléole interne, un point d'attache 72 destiné à fixer l'extrémité inférieure 6b du levier-ressort tibial 6 de l'appareillage selon l'invention. L'ouverture 71 permet un logement facile du pied et du cône inférieur jambier dans la coque 70. En outre, cette ouverture donne un degré de liberté, qui assure l'équilibre dynamique du mouvement du pied handicapé. A l'intérieur de la coque 70, sur la semelle 701, on dispose une semelle de soutien (non visible sur le dessin) moulée aux reliefs de la voûte plantaire du sujet en position d'équilibre vertical.

La chaussure selon l'invention comporte une armature insérée sur la coque et comprenant un levier-ressort 73 et des moyens élastiques régulateurs 74, 75 et 77.

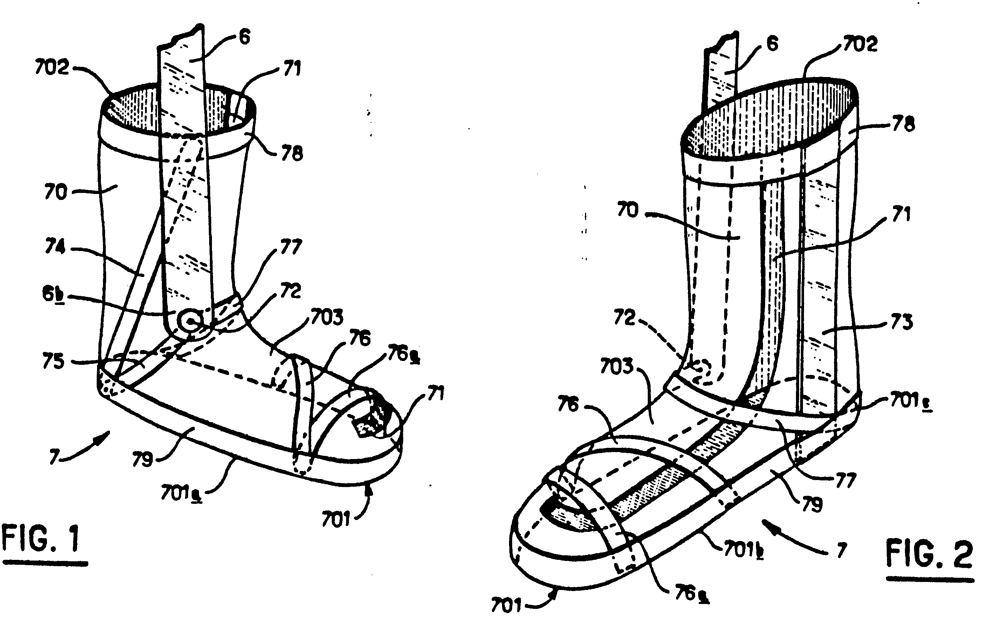

Le levier-ressort 73 est lié à la coque 70 au moins par ses deux extrémités. Dans la réalisation décrite, le levier-ressort 73 est collé extérieurement sur la coque 70 par ses deux extrémités; il est constitue d'une lame d'acier trempé de 40 mm de largeur sur 1 mm d'épaisseur. Dans une variante, on pourrait mettre en place ce levier-ressort en insert dans la coque 70 au moment du moulage de cette coque. Le levier-ressort 73 est disposé verticalement en regard du péroné depuis la face externe du calcanéum jusqu'à la zone de bordure supérieure 702 de la coque: il est visible sur la figure 1.

Les moyens élastiques régulateurs sont constitués d'une pluralité de bandes élastiques, qui compensent et régulent les actions du levier-ressort 73 et du levier-ressort tibial 6. Une bande élastique 74 est tendue depuis un point situé au niveau de la semelle 701 dans le cadran postéro-interne du talon jusqu'à la bordure supérieure 702 de la tige de la coque 70. La bande 74 fait un angle d'environ 30° avec la verticale; elle constitue un frein élastique régulant les mouvements de la face interne de la chaussure et favorisant les mouvements de vis interne. Une bande élastique 75 est disposée à partir du même point de la bordure interne 701a de la semelle 701; elle s'étend jusqu'au point d'implantation 72 du levier-ressort tibial 6 et constitue un frein élastique régulant l'action du levier-ressort 6 sur la chaussure 7. Une bande élastique 77 est tendue entre le point d'attache 72 du levier-ressort 6 et un point situé dans la zone postéro-externe 701c de la bordure externe 701b de la semelle 701; cette bande élastique 77 agit également comme un moyen de fermeture de la chaussure, puisqu'elle passe par-dessus l'ouverture 71 au niveau du coude-pied.

La chaussure 7 comporte, en outre, des moyens de fermeture, dont certains ont également un rôle de frein élastique au cours du mouvement. A cet égard, une bande élastique 76 est tendue entre un point situé sur la bordure interne 701a de la semelle 701, en regard du premier métatarsien, et un point situé sur la bordure externe 701b de la semelle 701, au niveau du bord externe du cuboïde; cette bande élastique 76 régule les mouvements de la partie supérieure 703 de l'empeigne de la chaussure et assure en même temps la fermeture de la chaussure, puisqu'elle est disposée transversalement par rapport à l'ouverture 71. On peut éventuellement, sans que cela soit obligatoire, mettre en place une autre bande élastique 76a, qui part du même point de la bordure interne 701a que la bande élastique 76 et qui va en un point de la bordure externe 701b de la semelle 701 situé en regard du cinquième métatarsien. La partie supérieure de la tige de la coque 70 est fermée par une bande de serrage 78 disposée tout le long de la bordure 702. Enfin, une bande 79 disposée tout autour de la semelle 701 enserre tous les points d'insertion de moyens élastiques disposés au niveau de la semelle 701. Toutes les bandes élastiques figurant dans la constitution de la chaussure 7 ont avantageusement une longueur réglable de façon à être ajustée aux caractéristiques du sujet, qui utilise l'appareillage.

On constate que l'utilisation d'une telle chaussure permet une légère flexion physiologique de la cheville au cours du déplacement du sujet ainsi qu'un déroulement du pas podal (talon-bord externe-bord interne).

**Revendications**

1. Chaussure orthopédique pour handicapés moteurs d'un (ou des deux) membre(s) inférieur(s), comprenant
- une coque semi-rigide (70) entourant le pied et le cône inferieur jambier, cette coque comportant une ouverture (71) qui s'étend en regard de la loge antéro-externe du cône jambier et en regard du bord externe du pied, au-dessus du cuboïde et d'au moins un rayon métatarsien;
- une armature élastique comportant, d'une part, un levier ressort (73) lié à la coque (70) et disposé verticalement en regard du péroné, depuis la face externe du calcanéum jusqu'à la zone de bordure supérieure (702) de la coque (70), et, d'autre part, des moyens élastiques régulateurs (74, 75, 77) implantés à l'extérieur de la coque, en premier lieu à partir du bord interne (701a) de la semelle (701) de la coque (70) vers la partie supérieure (703) de l'empeigne et vers la bordure superieure (702) de la tige de la coque (70), et, en second lieu, à partir de la zone de la malléole interne jusqu'à la zone postéro-externe (701c) du talon de la semelle (701);
- et des moyens de fermeture (76, 76a, 78) de la coque (70) de la chaussure.

2. Chaussure selon la revendication 1, caractérisée par le fait que les moyens de fermeture de la coque (70) comprennent un moyen élastique (76) tendu entre le bord interne (701a) de la semelle (701) de la coque (70), au niveau du premier métatarsien, et le bord externe (701b) de ladite semelle, au niveau du cuboïde.

3. Chaussure selon l'une des revendications 1 ou 2, caractérisée par le fait que les moyens de fermeture de la coque (70) comprennent un moyen élastique (76a) tendu entre le bord interne (701a) de la semelle (701) de la coque (70), au niveau du premier métatarsien, et le bord externe (701b) de ladite semelle, au niveau du cinquième métatarsien.

4. Chaussure selon l'une des revendications 1 à 3, caractérisée par le fait qu'en bout du pied, l'ouverture (71) de la coque (70) s'étend transversalement au-dessus du deux derniers métatarsiens.

5. Chaussure selon l'une des revendications 1 à 4, caractérisée par le fait que la coque (70) entoure le cône inférieur jambier jusqu'au dessus du tiers inférieur de la jambe et que les moyens de fermeture comprennent une bande de serrage (78) s'étendant tout le long de la bordure supérieure (702) de la tige de la coque (70).

6. Chaussure selon l'une des revendications 1 à 5, caractérisée par le fait que la coque (70) de la chaussure (7) est réalisee d'une seule pièce en matière plastique moulée.

7. Chaussure selon l'une des revendications 1 à 6, caractérisée par le fait que la chaussure (7) comprend intérieurement une semelle de soutien du pied, moulée aux reliefs de la voûte plantaire de l'utilisateur mis en position d'equilibre vertical.


**Patentansprüche**

1. Orthopädischer Schuh für Personen mit einem oder zwei motorisch behinderten unteren Gliedmaß(en), der aufweist:
- eine halbsteife Schale (70), welche den Fuß und den unteren Beinkonus umgibt, wobei diese Schale eine Öffnung (71) aufweist, die sich gegenüber der antero-externen Lage des Beinkonus und gegenüber dem Außenrand des Fußes oberhalb des Würfelbeines und mindestens eines metatarsalen Streifens erstreckt;
eine elastische Bewehrung, die einerseits eine Hebel-Federkombination (73), die mit der Schale (70) verbunden ist und vertikal gegenüber dem Wadenbein beginnend bei der Außenfläche des Fersenbeines und endend im Bereich des oberen Randes (702) der Schale (70) angeordnet ist, und andererseits elastische Regulierungsmittel (74, 75, 77) aufweist, die außen an der Schale erstens ausgehend vom Innenrand (701a) der Fußsohle (701) der Schale (70) zum oberen Teil (703) des Oberleders und zum oberen Rand (702) des Schaftes der Schale (70) und zweitens ausgehend vom Bereich des Malleoles tibiae zum postero-äußeren Bereich (701c) der Ferse der Fußsohle (701) angeordnet sind;
- und Verschlußmittel (76, 76a, 78) für die Schale (70) des Schuhes.

2. Schuh nach Anspruch 1, dadurch gekennzeichnet, daß die Verschlußmittel der Schale (70) ein elastisches Mittel (76) aufweisen, das zwischen dem Innenrand (701a) der Fußsohle (701) der Schale (70) im Bereich des ersten Metatarsalknochens und dem äußeren Rand (701b) dieser Fußsohle im Bereich des Würfelbeines gespannt ist.

3. Schuh nach Anspruch 1 oder 2, dadurch gekennzeichnet, daß die Verschlußmittel der Schale (70) ein elastisches Mittel (76a) aufweisen, das zwischen dem Innenrand (701a) der Fußsohle (701) der Schale (70) im Bereich des ersten Metatarsalknochens und dem äußeren Rand (701b) dieser Fußsohle im Bereich des fünften Metatarsalknochens gespannt ist.

4. Schuh nach einem der Ansprüche 1 bis 3, dadurch gekennzeichnet, daß sich die Öffnung (71) der Schale (70) am Fußende transversal oberhalb der beiden letzten Metatarsalknochen erstreckt.

5. Schuh nach einem der Ansprüche 1 bis 4, dadurch gekennzeichnet, daß die Schale (70) den unteren Beinkonus bis oberhalb des unteren Beindrittels umgibt und daß die Verschlußmittel ein Zugband (78) umfassen, das sich entlang des oberen Randes (702) des Schaftes der Schale (70) erstreckt.

6. Schuh nach einem der Ansprüche 1 bis 5, dadurch gekennzeichnet, daß die Schale (70) des Schuhes (7) einstückig aus einem Plastikmaterial geformt ist.

7. Schuh nach einem der Ansprüche 1 bis 6, dadurch gekennzeichnet, daß der Schuh (7) innen eine Fußstützsohle aufweist, die nach dem Fußgewölbe des in der vertikalen Gleichgewichtslage befindlichen Benutzern geformt ist.


**Claims**

1. An orthopaedic boot for persons with motor handicaps in one (or both) lower limbs, comprising
- a semi-rigid shell (70) surrounding the foot and the lower tibial conus, this shell comprising an opening (71) which extends opposite the antero-external location of the tibial conus and opposite the external edge of the foot, above the cuboid and at least one metatarsal zone;
- an elastic brace comprising on the one hand a spring lever (73) joined to the shell (70) and disposed vertically opposite the fibula from the external side of the calcaneus as far as the zone of the upper edge (702) of the shell (70), and, on the other hand, elastic regulating means (74, 75, 77) fitted outside the shell, firstly from the internal edge (701a) of the sole (701) of the shell (70) towards the upper portion (703) of the vamp and towards the upper edge (702) of the trunk of the shell (70), and secondly from the zone of the internal malleolus as far as the posteroexternal zone (701c) of the heel of the sole (701);
and closing means (76, 76a, 78) for the shell (70) of the boot.

2. A boot according to Claim 1, characterised in that the closing means for the shell (70) comprise an elastic means (76) stretched between the internal edge (701a) of the sole (701) of the shell (70), at the level of the first metatarsal and the external edge (701b) of the said sole at the level of the cuboid.

3. A boot according to one of Claims 1 or 2, characterised in that the closing means for the shell (70) comprise an elastic means (76a) stretched between the internal edge (701a) of the sole (701) of the shell (70) at the level of the first metatarsal and the external edge (701b) of the said sole, at the level of the fifth metatarsal.

4. A boot according to one of Claims 1 to 3, characterised in that at the foot end, the opening (71) of the shell (70) extends transversely above the last two metatarsals.

5. A boot according to one of Claims 1 to 4, characterised in that the shell (70) surrounds the lower tibial conus up to the top of the lower third of the leg and that the closing means comprise a tightening band (78) extending all along the upper edge (702) of the trunk of the shell (70).

6. A boot according to one of Claims 1 to 5, characterised in that the shell (70) of the boot (7) is made of a single piece of a moulded plastic material.

7. A boot according to one of Claims 1 to 6, characterised in that the boot (7) internally comprises a sole for supporting the foot, moulded to the contours of the plantar arch of the user, placed into the position of vertical equilibrium.

0 160 780

FIG. 2

FIG. 1